# EUROPEAN PATENT APPLICATION

(11) **EP 4 137 075 A1**
(43) Date of publication of application: **22.02.2023**
(21) Application number: 21306128.6
(22) Date of filing: 18.08.2021
(51) Int. Cl.: A61B 17/435, A61B 17/00

(54) **RECOVERABLE INTRA-UTERINE DEVICE**

(71) Applicant: Anecova S.A., 1015 Lausanne (CH)
(72) Inventor: KAMLAGE, Günter, 30419 HANNOVER (DE); SVALANDER, Peter, 1166 Perroy (CH); FABBRI, Kerstin, 1027 LONAY (CH); VELASCO, Martin, 1166 Perroy (CH)
(74) Representative: Santarelli

(57) **Abstract**

The invention, relates to a recoverable intra-uterine device comprising a housing (10), said housing (10) comprising an inside wall (11) formed from a biocompatible polymer, said inside wall (11) comprising at least one perforated part (13a, 13b), and an outside wall (12) covering said inside wall (11), the outside wall (12) being formed from a biocompatible silicone elastomer and comprising at least one opening (14a, 14b) facing said at least one perforated part (13a, 13b) of said inside wall (11).

The at least one perforated part (13a, 13b) of the inside wall (11) has perforations of dimensions comprised between 15 µm and 50 µm and has a thickness comprised between 40 µm and 80 µm.

## Description

The present invention concerns a recoverable intra-uterine device, used in particular for temporarily placing gametes and/or embryos in the uterus.

The present invention generally concerns the field of in-vivo and in-utero fecundation in assisted reproduction methods employing techniques of fertilization and/or preimplantation development.

Intra-uterine systems that are implantable and recoverable through the body's passages have been known for several years

Thus an intra-uterine device as described in document WO 03/011200 used in the in-vivo assistance in medically assisted reproduction processes is known.

Such a device is adapted to be placed in the uterine cavity for a period comprised between a few hours and a few days and enables the preimplantation development of an embryo.

The intra-uterine device comprises a housing adapted to contain the items to be encapsulated in the device, for example an embryo, male and/or female gametes, a fertilized oocyte, a non-fertilized ovum or for instance a combination of these items.

This encapsulation technique makes it possible to perform the fertilization and the development of an embryo inside a capsule placed in the uterus.

The Applicant has found that by transiently placing the capsule with an embryo in course of development in the uterus, the embryos so obtained were of better quality not only with regard to morphology but also with regard to their capacity to develop and to be re-implanted in the uterus later.

Thus, by obtaining embryos of better quality, it is possible to reduce the occurrences of multiple pregnancies by enabling the reimplantation of a single embryo with a greater chance of success than is obtained in the conventional methods of in-vitro assisted reproduction.

Furthermore, as the fertilization and preimplantation development take place directly in the uterus, the psychological repercussions on the couple may be high since the assisted reproduction process is closer to natural conception and requires higher implication by the couple.

The intra-uterine device makes it possible to place embryos in a natural environment during the preimplantation development phase, and thereby enables complex interactions between the endometrium of the uterus and the gametes and/or the embryos.

By minimizing the impact of the conditions of artificial culture (for example synthetic culture media) on the development of the embryo, the risk of alteration of the embryo by outside factors or by the lack of important substances for regulation and development is reduced.

In document WO 03/011200, the recoverable intra-uterine device comprises a housing of which the wall is formed from a porous permeable membrane, for example of polyether sulfone (PES).

The choice of such a porous membrane was initially guided to enable the protection of the gametes and/or embryos placed in the device while enabling the passage of nutrients, present in the uterine environment, into the housing of the device.

However, a porous permeable membrane is fragile and difficult to manipulate, in particular when loading and unloading the encapsulated items, or for instance when implanting and recovering the capsule inside the uterus.

It is indeed of the utmost importance not to deteriorate the capsule wall, which would induce the loss of the encapsulated items.

A recoverable intra-uterine device is also known from document US 2018/0310961 comprising a housing having an inside wall and an outside wall covering said inside wall, the inside wall comprising perforations of which the dimensions are comprised between 1 µm and 10 µm.

Even though this solution can be satisfying, it is desirable to facilitate even more the exchanges with the uterine matrix.

The present invention is thus directed to solving at least some of the aforementioned drawbacks and to provide a recoverable intra-uterine device promoting exchanges with the uterus, reliably and reproducibly, and with easy manipulation, without risk of altering the housing wall.

To that end, the invention relates to a recoverable intra-uterine device comprising a housing configured to contain one or more items chosen from the group comprising an embryo, male and/or female gametes, a fertilized oocyte, an unfertilized ovum and a combination of these items. The housing comprises an inside wall formed from a biocompatible polymer, said inside wall comprising at least one perforated part, and an outside wall covering said inside wall. The outside wall is formed from a biocompatible silicone elastomer and comprises at least one opening facing said at least one perforated part of said inside wall.

According to the invention, the inside wall has a thickness comprised between 40 µm and 80 µm, said at least one perforated part of the inside wall having perforations of dimensions comprised between 15 µm and 50 µm.

Thus, the housing comprises a double wall. The double wall is formed from the inside wall and the outside wall.

The perforated part of the inside wall forms a barrier between the inside of the housing, which is for containing gametes or an embryo, and the uterus in which the intra-uterine device is placed. The inside wall thus enables intra-uterine exchanges thanks to the perforations.

The inside wall is designed to facilitate the exchanges with the uterus. This is enabled by the combination between the thin inside wall and the relatively large perforations. In fact, such a combination provides perforations, which are short but large. The exchanges with the uterine matrix are thus facilitated and optimized.

Furthermore, the outside wall of the housing facilitates the manipulation of the housing by the biologist and/or the physician, by forming a protection made of a silicone elastomer.

The outside wall thus makes it possible to manipulate the housing without risk of deterioration or rupture of the inside wall, which is more fragile, comprising the perforated part to promote the exchanges with the uterus.

The inside wall of the recoverable intra-uterine device has the following features, alone or in combination with each other:
- the perforations have dimensions comprised between 17 µm and 35 µm;
- the inside wall has a thickness of 50 µm;
- the inside wall is formed from polyimide;
- the inside wall has at least one perforated part comprising between 10 000 perforation per cm² and 25 000 perforations per cm², preferably about 15 000 perforations ;
- the perforations are of substantially circular shape of diameter comprised between 15 µm and 50 µm, and preferably between 17 µm and 35 µm.

According to an example embodiment, the housing is of elongate cylindrical shape.

The outside wall comprises at least one opening extending in the longitudinal direction of the housing and over an angular sector of the housing comprised between 75° and 120°.

In practice, the inside wall comprises two perforated parts, which are diametrically opposite in the elongate cylindrical shape of the housing, and the outside wall comprises two openings respectively facing the two perforated parts of the inside wall.

The configuration of the housing results from a compromise between a high surface area for exchange with the uterus, at the location of the perforated parts of the inside wall, which are placed facing the openings of the outside wall, and good strength properties and manipulation of the intra-uterine device, thanks to the portions of outside wall.

In an application, the device contains at least one item chosen from the group comprising male and/or female gametes, a fertilized oocyte, a non-fertilized ovum or a combination of these items.

In another application, the device contains one or more embryos.

According to another aspect, the invention also concerns the use of the intra-uterine device for loading and/or unloading items chosen from the group comprising an embryo, male and/or female gametes, a fertilized oocyte, an unfertilized ovum and a combination of these items.

According to yet another aspect, the invention concerns a method of making the perforations of the inside wall of the device having the previously mentioned features. Said method comprises the following steps:
- providing an ultrashort pulse laser;
- focusing said laser to a point of the inside wall;
- generating multiple pulses to form by ablation at least one perforation around said point of the inside wall; and
- repeating the focusing step and the generating step at a different point as much as needed for producing the desired number of perforations.

By using ultrashort pulse laser, the deterioration of the thin and temperature sensitive inside wall is avoided. Indeed, the laser has thus only low thermal impact and the perforations can be created without burrs.

According to one embodiment, said laser output has a pulse width less than or equal to 300 femtoseconds, and preferably less than or equal to 200 femtoseconds.

According to one embodiment, the laser generates a beam having a Gaussian profile.

According to one embodiment, focusing of said laser is achieved by means of a lens.

According to one embodiment wherein the inside wall has a cylindrical shape, said laser is focused to a point of the inside wall and generates multiple pulses so as to form a first perforation and subsequently a second perforation, said second perforation being diametrically opposite to said first perforation.

This enables the creation of two perforations by focusing the laser on one same point. Forming perforations in the inside wall is thus simple and quick.

Still other particularities and advantages of the invention will appear in the following description.

In the accompanying drawings, given by way of non-limiting example:
- Figure 1 is a partial exploded perspective view of a recoverable intra-uterine device according to an embodiment of the invention;
- Figure 2 is a view in longitudinal section on line II-II of the recoverable intra-uterine device of Figure 1;
- Figure 3 is a cross-section view on line III - III of Figure 1;
- Figure 4 is a cross-section view diagrammatically illustrating the recoverable intra-uterine device according to an embodiment placed in a uterine cavity; and
- Figure 5 is an example profile of a laser beam generated from an ultrashort pulse laser used to create perforations in an inside wall of the intra-uterine device.

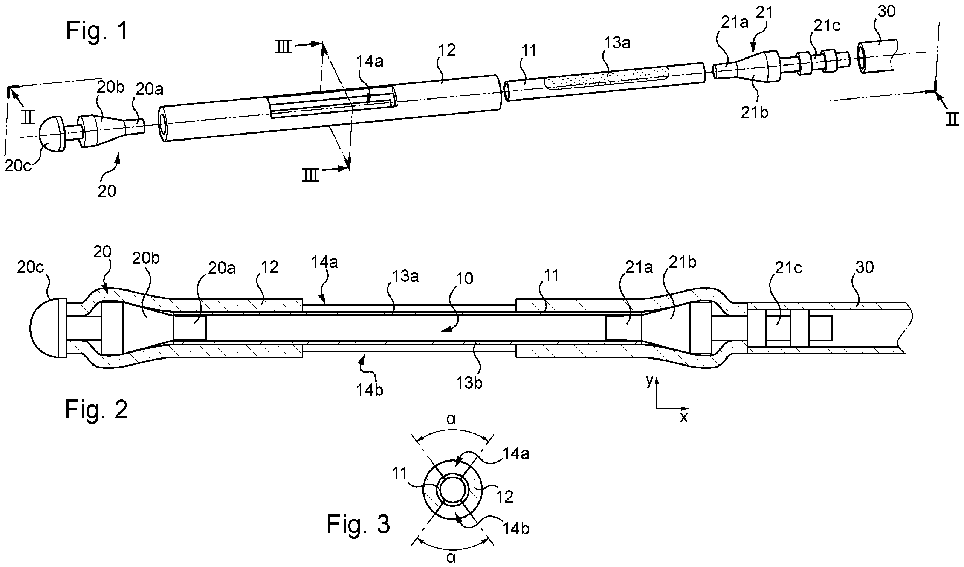

A description will first of all be made with reference to Figures 1 to 3 of an example embodiment of a recoverable intra-uterine device.

In general terms, the recoverable intra-uterine device comprises a housing 10 for containing various items employed in a process of medically assisted reproduction.

In particular, the housing 10 is configured to contain diverse items according to the state of progression of the fertilization process, for example male gametes and/or female gametes, an unfertilized ovum, or a fertilized oocyte, or one or more embryos.

It will be noted that the recoverable intra-uterine device is configured to contain one or other of these items according to the state of progression over time of the fertilization process.

The housing 10 is particular in that it comprises a double wall formed from an inside wall 11 and from an outside wall 12.

The inside wall 11 is formed from a biocompatible polymer.

As the inside wall 11 is adapted to form the inside cavity of the housing 10, it is important for the material used to be of medical quality and not to create conditions that are detrimental to the development of embryos.

By way of example, the inside wall 11 may be formed of polyimide.

In order to allow exchanges with the intra-uterine environment, the inside wall 11 comprises at least one perforated part 13a, 13b.

In this embodiment, and on a non-limiting basis, the housing is of cylindrical shape which is elongate in a longitudinal direction X.

Thus, the inside wall 11 of the housing 10 also has an elongate cylindrical shape.

In this embodiment, as clearly illustrated in Figure 2, the inside wall 11 comprises two perforated parts 13a, 13b which are diametrically opposite in the elongate cylindrical shape of the inside wall 11.

Of course, this embodiment is in no way limiting, and the inside wall could comprise a single perforated part or on the contrary, more than two perforated parts.

The Applicant has found that the perforations should be designed such as to promote the exchanges with the nutrients present in the uterine environment while enabling the protection of the items contained in the housing 10. In fact, the perforations should not be too small as they would not enable efficient exchanges. Neither should the perforations should be too large as this may be harmful for the items contained in the housing 10.

To that end, the perforations have dimensions comprised between 15 µm and 50 µm.

Such dimensions are sufficient and even ideal for the exchanges with the uterine matrix but prevent the items contained in the housing 10 from escaping into the uterus uncontrollably or detrimental cells from entering the housing 10.

The perforations may of course have various shapes.

Whatever its shape, such a perforation will have dimensions comprised between 15 µm and 50 µm provided that a circle circumscribing that perforation has a diameter comprised between 15 µm and 50 µm.

The perforations can have dimensions comprised between 15 µm and 45 µm.Preferably, the perforations have dimensions comprised between 15 µm and 40 µm, more preferably between 15 µm and 35 µm.

Even more preferably, the perforations have dimensions comprised between 17 µm and 35 µm.

As will appear below in the description of an embodiment of such a housing, the perforations may have a substantially circular shape of diameter comprised between 15 µm and 50 µm. For example, the diameter is comprised between 15 µm and 45 µm, preferably between 15 µm and 40 µm, more preferably between 15 µm and 35 µm, and even more preferably between 17 µm and 35 µm.

The perforations are preferably evenly disposed in the perforated part 13a, 13b.

The density of these perforations must be sufficient in the perforated part 13a, 13b to create an adequate surface area for exchange with the intra-uterine environment.

By way of example, the perforated part 13a, 13b comprises between 10 000 perforation per cm² and 25 000 perforations per cm². The perforations form for example 1% to 47% of the perforated part 13a, 13b.

The number of perforations depend on the dimensions of the inside wall 11.

Preferably, the perforated part 13a, 13b comprises 15 000 perforations per cm².

The Applicant has also found that the thickness of the inside wall 11 is also important in the quality of the exchanges of nutrients.

In particular, the inside wall 11 should be thin enough to enable quick exchanges with the uterine environment. Indeed, a thin inside wall 11 provides short perforations and thus a short path to be traveled by the nutrients from the uterine environment into the housing.

The inside wall 11 has a thickness between 40 µm and 80 µm. Preferably, the inside wall 11 has a thickness of 50 µm.

The choice of the material of the inside wall 11 is important for the thickness of the latter. Polyimide presents the advantage of enabling the manufacture of a thin inside wall 11.

The combination between a thin inside wall 11 and the dimensions of the perforations provides zones of exchanges with the uterine environment which are large enough and short (or thin). In other words, the nutrients can travel into the housing 10 through large zones via a short path. The exchanges are thus very efficient.

When the perforations have a circular shape, the perforations form thus large and short channels.

The outside wall 12 is formed from a biocompatible silicone elastomer.

Any type of silicone (or polymerized siloxane) taking the form of an elastomer may be used.

Other types of biocompatible elastomer may be used, for example a polyurethane thermoplastic elastomer or an elastomer of HCE type obtained by a hot curing process (HCE: initialism of Heat Curable Elastomer).

The outside wall 12 comprises at least one opening 14a, 14b for being positioned facing a perforated part 13a, 13b of the inside wall 11 when the outside wall 12 covers the inside wall 11.

In this embodiment, as the housing 10 has an elongate cylindrical shape, the outside wall 12 also has the general shape of an elongate cylinder.

On a non-limiting basis, in this embodiment in which the inside wall comprises two perforated parts 13a, 13b, the outside wall 12 comprises two openings 14a, 14b respectively facing the two perforated parts 13a, 13b of the inside wall 11.

Of course, the number and disposition of the openings 14a, 14b in the outside wall 12 are inherently linked to the number and the disposition of the perforated parts 13a, 13b of the inside wall 11.

In the embodiment described with reference to Figures 1 to 3, the outside wall 12 comprises two openings 14a, 14b which each extend in the longitudinal direction X of the housing 10.

As clearly illustrated in Figure 3, the two openings 14a, 14b are diametrically opposite in the elongate cylindrical shape of the outside wall 12.

Each opening 14a, 14b extends over an angular sector α of the housing 10 comprised between 75° and 120°. Preferably, each opening 14a, 14b extends over an angular sector α of 80° ± 5°.

For example, when the outside wall 12 comprises two openings 14a, 14b, the angular sector α of each of the openings is comprised between 75° and 90°.

In this embodiment, two openings 14a, 14b having an angular sector α of the same value have been illustrated by way of example.

Of course, the invention is not limited to this embodiment and the openings of the outside wall 12 can have angular sectors of different values.

In particular, an embodiment can be preferred in which the sum of the angular sectors α of the openings provided in the outside wall 12 remains less than 180° such that the outside wall 12 can maintain sufficient rigidity in the length of the housing 10, in particular to enable holding of the closure system.

Similarly, the length in the longitudinal direction X of each opening 14a, 14b is comprised between a quarter and half of the length of the outside wall 12 of the housing 10, and preferably substantially equal to one third of that length.

Preferably, the biocompatible polymer used to produce the inside wall 11 and the biocompatible silicone elastomer used to produce the outside wall 12 are transparent materials, enabling the biologist and/or the physician to observe the inside of the capsule when it is manipulated.

On a non-limiting basis, a dimensional example of a housing 10 of a recoverable intra-uterine device will be given.

The inside wall 11 may have an inside diameter of 350 µm and an outside diameter of 400 µm.

The thickness of the inside wall 11 is thus substantially equal to 50 µm.

The length in the longitudinal direction X of the inside wall 11 is comprised between 4 mm and 6 mm in length.

Correspondingly, the outside wall 12 has an inside diameter comprised between 300 µm and 500 µm and preferably equal to 430 µm.

The outside diameter of the outside wall 12 is comprised between 700 µm and 800 µm and is preferably equal to 790 µm.

The thickness of the outside wall 12 is thus comprised between 200 µm and 400 µm, and is preferably of 360 µm.

The length in the longitudinal direction X of the outside wall 12 is comprised between 7 mm and 8 mm.

The windows 14a, 14b thus extend in the longitudinal direction X over a length comprised between 2 mm and 3 mm, and preferably have a length equal to 2.8 mm.

The angular sector of each opening 14a, 14b is substantially equal to 80°.

Independently of the specific dimensions indicated above, the housing has an elongate form in the longitudinal direction X, the outside wall 12 extending beyond the ends of the inside wall 11 in the longitudinal direction X.

A housing 10 is thus obtained with an outside wall 12 forming a protective cage around the inside wall 11.

A description will now be given by way of non-limiting example of a method of producing such a double-walled housing 10.

A cylindrical tube of silicone elastomer is employed cut to length to form the outside wall 12.

The openings 14a, 14b may be produced for example by cutting with a laser or for instance by cutting with a water jet under pressure.

Perforations are made over a tubular portion of polymer, such as polyimide to constitute the inside wall 11.

The inside wall 11 is cut to length, then inserted into the outside wall 12.

The perforated parts of the inside wall 11 appear exposed through the openings 14a, 14b of the outside wall 12.

To that end, laser drilling technology can be used for the production of the perforations in the inside wall 11.

In particular, an ultrashort pulse laser is used for ablation of material from the inside wall 11 so as to create the perforations of the perforated parts 13a, 13b.

To create a perforation in the inside wall 11, the laser is focused to a point of the inside wall 11. The perforation is then formed by ablation of a part of the inside wall 11.

More precisely, multiple pulses are generated to form the perforation. The number of pulses generated by the laser is preferably less than or equal to 1000. The number of pulses generated by the laser can range for example between 100 and 200. More preferably, the number of pulses generated by the laser can be of 150.

The ultrashort pulse laser generates a laser output having a pulse width less than or equal to 300 femtoseconds. Preferably, the laser output has a pulse width less than or equal to 200 femtoseconds.

Ultrashort laser pulses prevent from creating heat which can destroy or damage the inside wall 11 since such technology has very low thermal impact compared to standard laser beams. Moreover, it is possible to form burr-free perforations.

This is very advantageous as the inside wall 11 which is made of a biocompatible polymer, e.g. polyimide, and which is very thin, is temperature sensitive.

The laser is focused on different points of the inside wall 11 for creating a plurality of perforations. Indeed, the focusing step is repeated at a different point of the inside wall 11 and multiple pulses are generated as much as needed for producing the desired number of perforations.

In the illustrated embodiment, the inside wall 11 has a cylindrical shape. In particular, the inside wall 11 is a tube. Thanks to the chosen parameters for the laser, two diametrically opposite perforations can be formed with the laser focused on the same point. In other words, the laser pulses focused on one point of the inside wall 11 form a first perforation around said point and subsequently a second perforation. The second perforation is diametrically opposite to the first perforation.

Preferably, to create the first perforation and the second perforation that is diametrically opposite to said first perforation, the number of pulses generated by the laser is of 150.

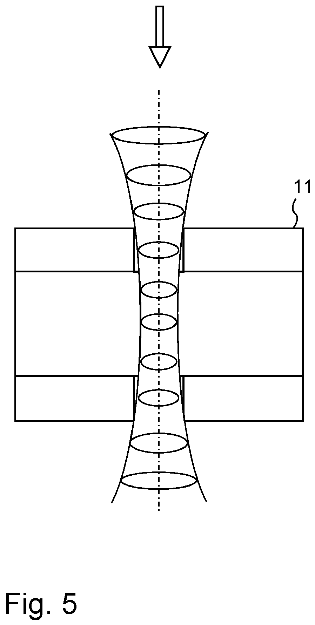

The laser pulses have a Gaussian profile as shown on figure 5.

The focusing of laser pulses is achieved by means of a single lens or multi-lens laser optic.

By controlling the laser parameters, it is possible to control the drilling of the perforations in the inside wall 11. In particular, it is possible to obtain perforations having the same dimensions, and without deterioration of the inside wall 11. By controlling the laser parameters, it is also possible to obtain perforations of the desired dimensions, for example of 25 µm.

The laser parameters that are controlled are for example: the pulse duration, the wavelength, the pulse repetition frequency, the beam diameter, the focal length, the pulse energy (or fluence), and the number of pulses.

The pulse width or pulse duration is the most important parameter to be set. As explained above, the pulse width is less than or equal to 300 femtoseconds, and is preferably less than or equal to 200 femtoseconds.

Perforations are thus obtained which are evenly distributed over the perforated part 13a, 13b of the inside wall 11.

A housing 10 is thus obtained formed from a double wall, taking the general shape of an elongate cylinder open at both its ends.

In order to obturate the housing, the intra-uterine device comprises at least one plug mounted at an end of the housing 10.

In this embodiment, the intra-uterine device comprises two plugs 20, 21 respectively mounted at the distal and proximal ends of the housing 10.

The plugs 20, 21 may for example be produced from titanium.

As clearly illustrated in Figure 2, each plug 20, 21 is mounted by friction at an end of the inside wall 11.

For this purpose, each plug 20, 21 comprises a first frusto-conical part 20a, 21a of which the outside diameter is, at its small base, slightly less than the inside diameter of the inside wall 11. The first frusto-conical part 20a, 21a widens to attain, at its larger base, an outside diameter slightly greater than the inside diameter of the inside wall 11.

Each first frusto-conical portion 20a, 21a is extended by a second frusto-conical portion 20b, 21b widening from the first frusto-conical portion 20a, 21a.

The second frusto-conical portion 20b, 21b is configured to be covered by one end of the outside wall 12 which extends beyond the ends of the inside wall 11, to an extender of the plug 20c, 21c.

The wider diameter of each plug 20, 21, and here the wide base of the second frusto-conical part 20b, 21b, has a dimension slightly greater than the inside diameter of the outside wall 12 such that the mounting of the plug 20, 21 is achieved by slight deformation of the outside wall 12 of the housing, providing perfect sealing for the mounting of the plug.

The distal plug 20 further comprises a first extender 20c having for example the shape of a nail head, enabling the physician or biologist to manipulate the distal plug 20 to enable the closing or the opening of the housing 10.

The proximal plug 21 is extended by a second extender 21c, forming a fastening stem 21c configured to be inserted by force into a connector 30 (partially illustrated in Figures 1 and 2).

Such a connector is described in particular in document FR 2 903 879 A1 and does not need to be described here in detail.

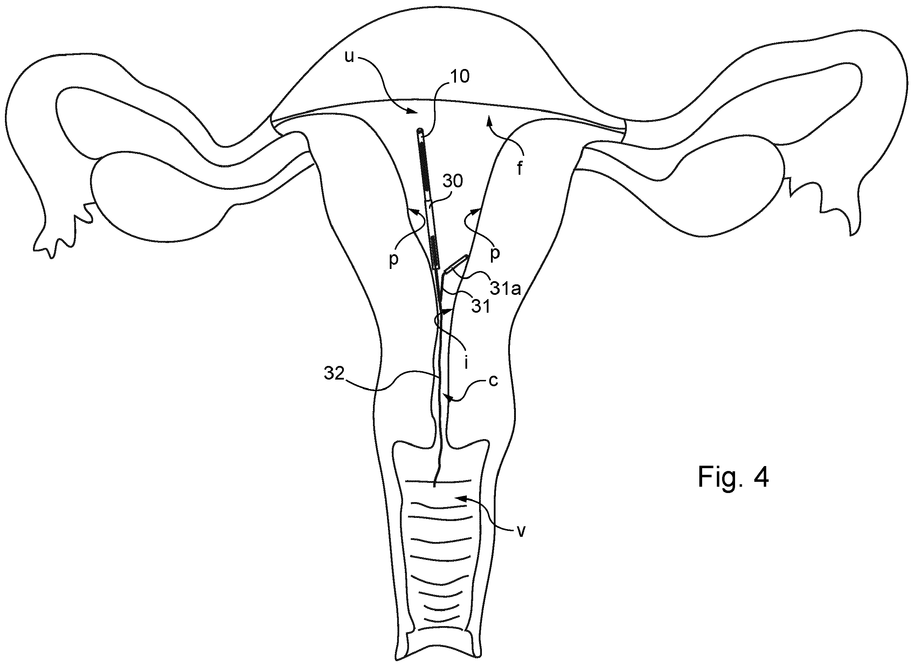

Only the essential parts of this connector are reviewed below with reference to Figure 4.

Such a connector is configured for mounting a stabilizing arm, useful for holding the intra-uterine device in position in the uterus, as well as for the mounting of a removal thread.

As clearly illustrated in Figure 4, the connector 30 comprises a cylindrical tube extending to the proximal end of the housing 10.

The cylindrical tube of the connector 30 is for example of silicone elastomer and is mounted by force at its distal end on the fastening stem 21c joined to the proximal plug 21.

A stabilizing arm 31 is fastened at the proximal end of the cylindrical tube of the connector 30.

In general terms, the stabilizing arm 31 can adopt a folded position, thus extending in the longitudinal direction X of the housing 10 and of the connector 30, and an extended position such as illustrated in Figure 4 in which, by a spring effect, the stabilizing arm 31 unfolds, one end 31a of the arm coming locally into contact with the uterus u.

The stabilizing arm 31 can for example be formed of steel and may have at its end 31a a protective sleeve of silicone elastomer.

Furthermore, a removal thread 32 of nylon to enable the removal of the device from the uterus by pulling is fastened to the proximal end of the cylindrical tube of the connector 30.

The principle of use and of putting in place the intra-uterine device is similar to that described in document FR 2 903 879 A1

As clearly illustrated in Figure 4, the intra-uterine device is inserted through the body's passages into the uterus by means of a transfer catheter (not shown).

The transfer catheter is next withdrawn enabling the stabilizing arm 31 to extend and hold the intra-uterine device in place for a few hours or a few days, enabling preimplantation development of the embryo in the intra-uterine environment.

Thanks to the porosity of the inside wall 11 of the housing 10, at the location of the perforated parts 13a, 13b, exchanges between the gametes and/or embryos contained in the intra-uterine device and the uterine fluids are promoted and enable optimal development of the embryo.

To that end, the intra-uterine device is placed in the uterus u near the fundus f and the end 31a of the stabilizing arm 31 comes into contact only with a very small portion of the endometrial wall at the location of the cervical canal i.

Thus, the endometrial layer that lines the uterine cavity at the fundus f, the corpus p and the cervical canal i have very little contact with the intra-uterine device, avoiding any deterioration of the endometrium which could be detrimental to the later reimplantation of the embryo.

Furthermore, the removal thread 32 extends through the cervix c to emerge in the vagina v and enable easy extraction of the device by the practitioner, simply by pulling.

Of course, the invention is not limited to the embodiments described above and numerous modifications may be made to those examples without departing from the scope of the invention.

## Claims

1. A recoverable intra-uterine device comprising a housing (10) configured to contain one or more items chosen from the group comprising an embryo, male and/or female gametes, a fertilized oocyte, an unfertilized ovum and a combination of these items, said housing (10) comprising an inside wall (11) formed from a biocompatible polymer, said inside wall (11) comprising at least one perforated part (13a, 13b), and an outside wall (12) covering said inside wall (11), the outside wall (12) being formed from a biocompatible silicone elastomer and comprising at least one opening (14a, 14b) facing said at least one perforated part (13a, 13b) of said inside wall (11), the device being **characterized in that** said inside wall has a thickness comprised between 40 µm and 80 µm, said at least one perforated part (13a, 13b) of the inside wall (11) having perforations of dimensions comprised between 15 µm and 50 µm.

2. A device according to claim 1, **characterized in that** said perforations have dimensions comprised between 17 µm and 35 µm.

3. A device according to one of claims 1 or 2, **characterized in that** the inside wall (11) has a thickness of 50 µm.

4. A device according to one of claims 1 to 3, **characterized in that** said inside wall (11) is formed from polyimide.

5. A device according to one of claims 1 to 4, **characterized in that** said at least one perforated part (13a, 13b) of the inside wall (11) comprises between 200 and 400 perforations, preferably 300 perforations.

6. A device according to one of claims 1 to 5, **characterized in that** the perforations are of substantially circular shape of diameter comprised between 15 µm and 50 µm, and preferably between 17 µm and 35 µm.

7. A device according to one of claims 1 to 6, **characterized in that** said housing (10) is of elongate cylindrical shape.

8. A device according to claim 7, **characterized in that** the outside wall (12) comprises at least one opening (14a, 14b) extending in the longitudinal direction (X) of the housing (10) and over an angular sector (α) of the housing (10) comprised between 75° and 120°.

9. A device according to one of claims 7 or 8, **characterized in that** the inside wall (11) comprises two perforated parts (13a, 13b), which are diametrically opposite in said elongate cylindrical shape of the housing (10), and the outside wall (12) comprises two openings (14a, 14b) respectively facing said two perforated parts (13a, 13b) of the inside wall (11).

10. A device according to one of claims 1 to 9, **characterized in that** said device contains at least one item chosen from the group comprising male and/or female gametes, a fertilized oocyte, a non-fertilized ovum or a combination of these items.

11. A device according to one of claims 1 to 10, **characterized in that** said device contains one or more embryos.

12. A method of making the perforations of the inside wall (11) of the device (1) according to any one of claims 1 to 11, said method comprising the following steps:
- providing an ultrashort pulse laser;
- focusing said laser to a point of the inside wall (11);
- generating multiple pulses to form by ablation at least one perforation around said point of the inside wall; and
- repeating the focusing step and the generating step at a different point as much as needed for producing the desired number of perforations.

13. A method of making the perforations of the inside wall according to claim 12, wherein the ultrashort pulse laser generates a laser output having a pulse width less than or equal to 300 femtoseconds, and preferably less than or equal to 200 femtoseconds.

14. A method of making the perforations of the inside wall according to claim 12 or claim 13, wherein the laser generates a beam having a Gaussian profile.

15. A method of making the perforations of the inside wall according to any one of claims 12 to 14, wherein the inside wall (11) has a cylindrical shape, and wherein said laser is focused to a point of the inside wall (11) and generates multiple pulses so as to form a first perforation and subsequently a second perforation, said second perforation being diametrically opposite to said first perforation.
